# EUROPÄISCHE PATENTSCHRIFT

(11) **EP 4 312 467 B1**
(45) Veröffentlichungstag und Bekanntmachung des Hinweises auf die Patenterteilung: **18.09.2024**
(21) Anmeldenummer: 22187531.3
(22) Anmeldetag: 28.07.2022
(51) Int. Cl.: H05G 1/02, H05G 1/70, A61B 6/03, A61B 6/40, A61B 6/02, A61B 6/50, A61B 6/00

(54) **RÖNTGENSTRAHLERGEHÄUSE MIT ZUMINDEST EINEM ELEKTRISCH LEITFÄHIGEN GEHÄUSEABSCHNITT**
X-RAY GENERATOR CASING WITH AT LEAST ONE ELECTRICALLY CONDUCTIVE CASING SECTION
BOÎTIER D'ÉMETTEUR DE RAYONS X DOTÉ D'AU MOINS UNE SECTION DE BOÎTIER ÉLECTRIQUEMENT CONDUCTRICE

(43) Veröffentlichungstag der Anmeldung: 31.01.2024
(73) Patentinhaber: Siemens Healthineers AG, 91301 Forchheim (DE)
(72) Erfinder: Freudenberger, Jörg, 90562 Kalchreuth (DE); Schäff, Wolfgang, 90559 Burgthann (DE)
(74) Vertreter: Siemens Healthineers Patent Attorneys

(56) Entgegenhaltungen:
- US-A1- 2022 208 504

## Beschreibung

Die Erfindung betrifft ein Multiröhren-Röntgenstrahlergehäuse, eine Multiröhren-Röntgenstrahlenquelle, eine Röntgen-Einrichtung und eine Computertomographie-Einrichtung.

Eine typische Röntgenröhre einer Röntgenstrahlenquelle kann lediglich in einem Brennfleck mit einer Fläche im Millimeter- oder niedrigen Zentimeterbereich Röntgenstrahlen generieren. Da bei einigen Anwendungen von bildgebenden Systemen eine Durchleuchtung eines größeren Untersuchungsbereichs mittels der Röntgenstrahlen erfolgen soll, ist regelmäßig eine mitunter aufwändige Mechanik vonnöten, um eine einzelne oder auch zwei einzelne Röntgenröhren zur Durchleuchtung des größeren Untersuchungsbereichs zu verfahren.

Bildgebende Systeme mit verteilten Röntgenstrahlenquellen sind insbesondere dadurch charakterisiert, dass die Röntgenstrahlenquellen, insbesondere eine Vielzahl davon, im Raum verteilt angeordnet sind. Bei einem solchen bildgebenden System fällt insbesondere die Notwendigkeit der Mechanik zum Verfahren einzelner Röntgenröhren weg. Der größere Untersuchungsbereich kann dann insbesondere durch die im Raum verteilten Röntgenstrahlenquellen ohne Verfahrmechanik durchleuchtet werden. Insbesondere für ein als Computertomographie-Einrichtung ausgebildetes bildgebendes System kann es darauf ankommen, dass die Brennflecke auf den Anoden einen möglichst geringen Abstand und einen möglichst gleichbleibenden Abstand aufweisen. Insbesondere der geringe Abstand ist aufgrund der Hochspannung führenden Elektroden in der Röntgenröhre oder der Hochspannung führenden Hochspannungskontakten technisch anspruchsvoll.

EP 3 586 752 A1 offenbart ein statisches bildgebendes System mit einer multifokalen ringförmigen Röntgenstrahlenquelle und mit einem ringförmigen Photondetektor, wobei die Röntgenstrahlenquelle aus einer Vielzahl an Röntgenröhren in einer ringförmigen Anordnung besteht und wobei jede Röntgenröhre einen breiten auf das Untersuchungsobjekt ausgerichteten Röntgenstrahl generiert. Typisch für dieses bildgebende System ist regelmäßig, dass die Röntgenröhren gegenseitig hochspannungsisoliert sein müssen und jede Röntgenröhre eine eigene individuelle Hochspannungsversorgung aufweist.

In US 11 183 357 B2 ist eine Multiröntgenstrahlen-Röntgenröhre offenbart, welche in einem evakuierten Gehäuse eine als Kühlfinger ausgestatte Anode aufweist, wobei das Gehäuse eine Vielzahl an Kathodenzuführungen und nicht mehr als zwei Hochspannungsbuchsen aufweist und wobei in der Hochspannungsbuche ein flüssiges Kühlmedium fließen kann. Typisch für ein solches bildgebendes System ist insbesondere, dass alle Seitenflächen des evakuierten Gehäuses auf Erdpotential liegen und ein Isolationsabstand zwischen der Anode oder der Kathode und der Seitenfläche einzuhalten ist. Daraus folgt typischerweise, dass ein Abstand zwischen mehreren Multiröntgenstrahlen-Röntgenröhren relativ groß ist und ein Abstand zwischen Brennflecken auf der Anode variieren kann. Ein weiterer Nachteil betrifft den vergleichsweise komplexen Aufbau, welcher insbesondere im Falle einer oder mehrerer defekter Kathoden einen kompletten Austausch der Multiröntgenstrahlen-Röntgenröhre mit sich bringen kann.

Der Erfindung liegt die Aufgabe zu Grunde, ein Multiröhren-Röntgenstrahlergehäuse, eine Multiröhren-Röntgenstrahlenquelle, eine Röntgen-Einrichtung und eine Computertomographie-Einrichtung anzugeben, bei welchen die Röntgenröhren gleichmäßiger verteilt angeordnet und einfacher ausgetauscht werden können.

Die Aufgabe wird durch die Merkmale der unabhängigen Ansprüche gelöst. Vorteilhafte Ausgestaltungen sind in den Unteransprüchen beschrieben.

Das erfindungsgemäße Multiröhren-Röntgenstrahlergehäuse weist
- ein Gehäuse mit zumindest einer Seitenfläche,
- eine Hochspannungsversorgung und
- eine Kühleinrichtung mit einem elektrisch isolierenden Kühlmedium auf,

wobei die Hochspannungsversorgung innerhalb des Gehäuses eine einzelne Hochspannungszuführung zur Führung einer Hochspannung und mehrere an der einzelnen Hochspannungszuführung parallel geschaltete Hochspannungskontakte aufweist,
wobei die Hochspannungskontakte jeweils mit einer Hochspannungselektrode zur Bereitstellung der Hochspannung koppelbar sind und auf demselben Hochspannungspotential liegen,
wobei eine erste der zumindest einen Seitenfläche einen ersten elektrisch leitfähigen Gehäuseabschnitt mit einer temperaturabhängigen elektrischen Leitfähigkeit aufweist,
wobei sich zwischen dem ersten elektrisch leitfähigen Gehäuseabschnitt und einem der Hochspannungskontakte elektrisch isolierendes Kühlmedium befindet,
gekennzeichnet
durch eine Regeleinheit, welche eine Schnittstelle zum Empfang eines die elektrische Leitfähigkeit des ersten elektrisch leitfähigen Gehäuseabschnitts abbildenden Messwerts aufweist und zum Vergleichen des Messwerts mit einem Schwellwert ausgebildet ist, und
durch eine Schalteinrichtung zur Abschaltung der Hochspannung in Abhängigkeit des Vergleichsergebnisses.

Der erste elektrisch leitfähige Gehäuseabschnitt bewirkt, dass beim Anlegen einer Hochspannung typischerweise ein Strom über diesen Gehäuseabschnitt fließt. Dadurch stellt sich vorteilhafterweise ein im Wesentlichen linearer Potentialverlauf entlang des ersten elektrisch leitfähigen Gehäuseabschnitts ein. Insbesondere gestreute Elektronen können vorzugsweise über den ersten elektrisch leitfähigen Gehäuseabschnitt abgeleitet werden und können somit elektrische Aufladungen reduzieren oder verhindern. Ein weiterer Vorteil betrifft demnach die Verringerung an Kriechstrecken und/oder an Abschirmeinrichtungen für elektrische Aufladungen im ersten elektrisch leitfähigen Gehäuseabschnitt.

Ein Vorteil des Multiröhren-Röntgenstrahlergehäuses ist, dass im Vergleich zu einem herkömmlichen Gehäuse zumindest eine Seitenfläche nicht aus (hoch-)isolierenden Bauteilen bestehen muss, um die Hochspannung zwischen einem der Hochspannungskontakte und dem Gehäuse zu isolieren. Vorzugsweise können dadurch, dass die zumindest eine Seitenfläche einen ersten elektrisch leitfähigen Gehäuseabschnitt aufweist, herkömmliche Isolationsmaßnahmen wie z.B. ein entsprechender Isolationsabstand zumindest teilweise abgeschwächt oder vollkommen ersetzt werden.

Das Multiröhren-Röntgenstrahlergehäuse mit dem ersten elektrisch leitfähigen Gehäuseabschnitt ermöglicht vorteilhafterweise, dass die Hochspannungskontakte insgesamt näher an dem Gehäuse und somit auch an weiteren Hochspannungskontakten eines weiteren Multiröhren-Röntgenstrahlergehäuses angeordnet sein kann.

Das Multiröhren-Röntgenstrahlergehäuse kann grundsätzlich Eintankgehäuse oder Monoblockgehäuse genannt werden. Das Multiröhren-Röntgenstrahlergehäuse ist insbesondere ein Gehäuse, welches grundsätzlich dazu geeignet ist, mehrere Röntgenröhren zumindest teilweise aufzunehmen und/oder die Hochspannungsversorgung für die mehreren Röntgenröhren zu enthalten
Das Multiröhren-Röntgenstrahlergehäuse weist regelmäßig keine Röntgenstrahlengenerierende Bauteile, wie z.B. die mehreren Röntgenröhren auf. In anderen Worten sind keine Röntgenröhren oder andere Röntgenstrahlengenerierende Bauteile mit dem Multiröhren-Röntgenstrahlergehäuse zwingend gekoppelt, sie sind allerdings koppelbar. Das Multiröhren-Röntgenstrahlergehäuse kann insbesondere mehrere Röntgenröhren koppeln, um mittels der geführten Hochspannung Röntgenstrahlen zu generieren, und kann insbesondere ohne die mehreren Röntgenröhren keine Röntgenstrahlen generieren.

Die Hochspannungsversorgung ist zumindest teilweise innerhalb des Gehäuses angeordnet. Die Hochspannungsversorgung weist insbesondere einen Hochspannungserzeuger auf, welcher innerhalb oder außerhalb des Gehäuses angeordnet sein kann. Der Hochspannungserzeuger stellt insbesondere die Hochspannung an einem Ausgang in Abhängigkeit einer an einem Eingang angelegten Nieder- oder Netzspannung bereit. Dafür kann der Hochspannungserzeuger einen Transformator und/oder einen Gleichrichter umfassen. Wenn der Hochspannungserzeuger innerhalb des Gehäuses angeordnet ist, kann die Nieder- oder Netzspannung beispielsweise über einen in das Gehäuse integrierten Stecker von außen in das Gehäuse hinein an den Eingang des Hochspannungserzeugers hinzugeführt sein. Alternativ kann der Stecker ein Hochspannungsstecker sein, wenn der Hochspannungserzeuger außerhalb des Gehäuses angeordnet ist.

Die Hochspannung ist insbesondere eine Gleichspannung und/oder beträgt beispielsweise zwischen 20 und 200 kV, insbesondere mehr als 40 kV und/oder weniger als 150 kV. Die Hochspannungsversorgung nimmt typischerweise die an dem Ausgang bereitgestellte Hochspannung ab und führt die abgenommene Hochspannung in der einzelnen Hochspannungszuführung. Die Hochspannung dient insbesondere einer Beschleunigung von Elektronen innerhalb einer Röntgenröhre.

Die einzelne Hochspannungszuführung stellt insbesondere sicher, dass die mehreren Hochspannungskontakte dieselbe Hochspannung führen. Dadurch ist insbesondere eine Komplexität der Hochspannungsversorgung reduziert, weil die parallel geschalteten Hochspannungskontakte auf demselben Hochspannungspotential liegen. Die mehreren Hochspannungskontakte umfassen mindestens zwei Hochspannungskontakte, insbesondere einen ersten Hochspannungskontakt und einen zweiten Hochspannungskontakt. Die einzelne Hochspannungszuführung kann ein Hochspannungskabel und/oder eine Platine umfassen. Die einzelne Hochspannungszuführung kann elektrisch isoliert ausgebildet sein, beispielweise durch eine entsprechende Abschirmung des die Hochspannung führenden Teils.

Dass die Hochspannungskontakte jeweils mit einer Hochspannungselektrode koppelbar sind, bedeutet insbesondere, dass eine elektrisch leitfähige Verbindung zwischen einem Hochspannungskontakt und einer Hochspannungselektrode nur dann besteht, wenn sie miteinander gekoppelt sind. In anderen Worten entsteht erst durch die erfolgte Kopplung eine elektrisch leitfähige Verbindung. Die Hochspannung liegt typischerweise an einer der Hochspannungselektroden an, wenn diese Hochspannungselektrode mit dem jeweiligen Hochspannungskontakt gekoppelt ist. Wenn eine Hochspannungselektrode nicht mit einem Hochspannungskontakt gekoppelt ist, liegt an so einer Hochspannungselektrode insbesondere nicht die Hochspannung an. Die Hochspannungskontakte sind regelmäßig nicht vollständig elektrisch isoliert, um eine Kopplung zu ermöglichen. Die Hochspannungskontakte sind insbesondere kontaktierbar, beispielsweise von einer Hochspannungselektrode.

Das Gehäuse weist zumindest eine Seitenfläche auf. Das Gehäuse bildet insbesondere einen Container, in welchem die Hochspannungsversorgung und/oder die koppelbaren Röntgenröhren enthalten sein können. Die zumindest eine Seitenfläche umgibt z.B. die Hochspannungsversorgung und/oder das elektrisch isolierende Kühlmedium räumlich. Das Gehäuse kann typischerweise mehrere Seitenflächen aufweisen. Ein Teil der Seitenflächen kann miteinander fest verbunden sein und/oder einstückig ausgebildet sein. Es ist denkbar, dass eine Seitenfläche mittels eines Scharniers an eine andere Seitenfläche befestigt ist und beispielsweise einen Deckel des Gehäuses bildet. Im Übrigen umfasst der Begriff Seitenfläche in diesem Fall insbesondere ausdrücklich eine Oberseite und/oder eine Unterseite des Gehäuses. Mit Seitenfläche ist in anderen Worten jede Seite des Gehäuses unabhängig von der Ausrichtung des Gehäuses gemeint.

Die zumindest eine Seitenfläche kann einen planen, einen gekrümmten oder einen eine Oberfläche vergrößernde Struktur aufweisenden Gehäuseabschnitt aufweisen. Die die Oberfläche vergrößernde Struktur umfasst beispielsweise Kühlrippen oder Kühlfinnen und kann insbesondere als besonders geeignete Kühlvorrichtung wirken, welche in Hinblick auf einen planen Gehäuseabschnitt oder einen gekrümmten Abschnitt verbesserte Kühleigenschaften aufweist. Es ist denkbar, dass eine Seitenfläche mehrere Gehäuseabschnitte aufweist, welche identisch oder verschieden in Hinblick auf deren Materialzusammensetzung und/oder Oberflächenbeschaffenheit ausgebildet sein können. Die Seitenfläche kann grundsätzlich aus einem elektrisch leitfähigen Gehäuseabschnitt bestehen oder einen solchen einrahmen. Der Rahmen kann beispielsweise aus einem elektrisch isolierenden Material ausgestaltet sein.

Eine zweite Seitenfläche des Gehäuses kann einen zweiten elektrisch leitfähigen Gehäuseabschnitt aufweisen. Der zweite elektrisch leitfähige Gehäuseabschnitt kann derart ausgebildet sein, dass dieser Gehäuseabschnitt dieselbe elektrische Leitfähigkeit aufweist wie der erste elektrisch leitfähige Gehäuseabschnitt. Der erste elektrisch leitfähige Gehäuseabschnitt und der zweite elektrisch leitfähige Gehäuseabschnitt sind typischerweise auf gegenüberliegenden Seiten des Gehäuses angeordnet. Übrige Seitenflächen des Gehäuses können insbesondere elektrisch isolierend ausgebildet sein. In eine Seitenfläche des Gehäuses ist regelmäßig ein Röntgenstrahlenaustrittfenster integriert.

Der erste elektrisch leitfähige Gehäuseabschnitt weist eine temperaturabhängige elektrische Leitfähigkeit auf, welche insbesondere von dem Material oder der Materialzusammensetzung des Gehäuseabschnitts und regelmäßig von der Temperatur abhängt. Im Allgemeinen können Materialien in Abhängigkeit ihrer jeweiligen elektrischen Leitfähigkeit unterschieden und grob in die Klassen Isolatoren (Nichtleiter), Halbleiter, Leiter oder Supraleiter eingeteilt werden. Ein Übergang zwischen den Klassen ist typischerweise fließend. Das Material und/oder die Materialzusammensetzung des ersten elektrisch leitfähigen Gehäuseabschnitts kann insbesondere bei einer ersten Temperatur isolierend, bei einer höheren Temperatur "wenig" leitend und bei einer erneut höheren Temperatur "normal" leitend sein.

Der erste elektrisch leitfähige Gehäuseabschnitt ist insbesondere nicht-perfekt-isolierend oder schwach-leitend bei der Betriebstemperatur ausgebildet. Das Material bzw. die Materialzusammensetzung des Gehäuseabschnitts kann gemäß den Definitionen in https://de.wikipedia.org/wiki/Elektrische_Leitfähigkeit der Klasse Isolator oder Nichtleiter zugeordnet sein, wird vorzugsweise allerdings insbesondere einer der Klassen Halbleiter, Leiter oder Supraleiter angehören. Typischerweise kann der erste elektrisch leitfähige Gehäuseabschnitt derart ausgestaltet sein, dass die elektrische Leitfähigkeit bei einem unteren Temperaturschwellwert mindestens 10^-8 S/m und/oder bei einem oberen Temperaturschwellwert maximal 10^-4 S/m beträgt. Gemäß Wikipedia ist der erste elektrisch leitfähige Gehäuseabschnitt nicht ein sogenannter guter Isolator. Ein Stromfluss entlang des ersten elektrisch leitfähigen Gehäuseabschnitts ist insbesondere bewusst zugelassen, vorzugsweise ab einem minimalen Amplitudenwert und/oder bis zu einem maximalen Amplitudenwert erwünscht. Die über den ersten elektrisch leitfähigen Gehäuseabschnitt abgeführte Ladungsmenge wird insbesondere an einen Masseanschluss und/oder an einen Schutzleiter abgeführt. Der erste elektrisch leitfähige Gehäuseabschnitt wirkt typischerweise als Kondensator und/oder es wird die Ladungsmenge durch den ersten elektrisch leitfähigen Gehäuseabschnitt hindurch oder an seiner Oberfläche abtransportiert, z.B. in Richtung der Kathode oder eines Kathodenteils, welches in einem kurzen Abstand von diesem Gehäuseabschnitt liegt. Der elektrische Strom, sprich die Ladungsmenge, fließt beispielsweise durch das elektrisch isolierende Kühlmedium und/oder zu einem zweiten elektrisch leitfähigen Gehäuseabschnitt.

Der untere Temperaturschwellwert beträgt beispielsweise mindestens -50 C°, vorzugsweise mehr als 0 C°. Der obere Temperaturschwellwert beträgt insbesondere maximal 500 C°, vorzugsweise weniger als 100 C°. Die Betriebstemperatur liegt insbesondere zwischen dem unteren Temperaturschwellwert und dem oberen Temperaturschwellwert. Die Betriebstemperatur kann insbesondere einer Raumtemperatur oder einem Temperaturfenster von 5°C als unteren Temperaturschwellwert bis 25°C als oberen Temperaturschwellwert, vorzugsweise 20° bis 25° gemäß den Angaben in der Wikipedia, entsprechen. Das Material oder die Materialzusammensetzung weist insbesondere eine Durchschlagsspannung auf, welche höher ist als die Hochspannung.

Die Kühleinrichtung weist das elektrisch isolierende Kühlmedium auf. Das elektrisch isolierende Kühlmedium ist insbesondere kein Vakuum. Das elektrisch isolierende Kühlmedium ist insbesondere ein Fluid, insbesondere flüssig und/oder gasförmig. Das elektrisch isolierende Kühlmedium ist vorzugsweise innerhalb des Gehäuses angeordnet, kann grundsätzlich alternativ oder zusätzlich das Gehäuse umströmen. Das Gehäuse kann insbesondere fluiddicht ausgebildet sein.

Dass sich zwischen dem ersten elektrisch leitfähigen Gehäuseabschnitt und einem der Hochspannungskontakte elektrisch isolierendes Kühlmedium befindet, bedeutet insbesondere, dass das Gehäuse so viel Kühlmedium umfasst, dass das Kühlmedium wenigstens die direkte Sichtverbindung zwischen dem ersten elektrisch leitfähigen Gehäuseabschnitt und einem der Hochspannungskontakte abdeckt. Dafür kann auch dieser Hochspannungskontakt und der erste elektrisch leitfähige Gehäuseabschnitt entsprechend innerhalb des Gehäuses positioniert sein. Vorteilhafterweise ist ein Abstand zwischen dem ersten elektrisch leitfähigen Gehäuseabschnitt und einem der Hochspannungskontakte geringer als eine Isolationsstrecke entlang des elektrisch isolierenden Kühlmediums. In anderen Worten ist eine Isolationsfähigkeit des Kühlmediums und/oder die Menge und/oder Volumen an Kühlmedium zwischen dem ersten elektrisch leitfähigen Gehäuseabschnitt und einem der Hochspannungskontakte zu gering, um die Isolation und/oder Durchschlagfestigkeit im Betrieb des Multiröhren-Röntgenstrahlergehäuses zu gewährleisten. Die Isolation gegen die Hochspannung wird vorteilhafterweise zumindest teilweise durch den linearen Potentialverlauf entlang des ersten elektrisch leitfähigen Gehäuseabschnitts übernommen, so dass der Betrieb des Multiröhren-Röntgenstrahlergehäuse regulatorisch zulässig ist.

Das Gehäuse kann grundsätzlich vollständig mit dem Kühlmedium gefüllt sein. In diesem Fall weist das Multiröhren-Röntgenstrahlergehäuse innerhalb des Gehäuses typischerweise ein Ausdehnungsausgleichgefäß und/oder ein Druckventil auf.

Das Multiröhren-Röntgenstrahlergehäuse weist insbesondere die Regeleinheit und die Schalteinrichtung auf. Die Regeleinheit kann den Messwert empfangen und insbesondere verarbeiten. Das Verarbeiten umfasst beispielsweise das Vergleichen des Messwerts mit dem Schwellwert. Das Verarbeiten, insbesondere das Vergleichen, des Messwerts kann gemäß Programmodemitteln erfolgen. Die Regeleinheit kann einen Rechen- oder Logikbaustein zur Ausführung der Programmcodemittel und/oder eine Speichereinheit zur Speicherung und/oder zum Bereitstellen des Schwellwerts aufweisen. Beim Vergleichen des Messwerts mit dem Schwellwert wird insbesondere das Vergleichsergebnis berechnet oder ermittelt. Die Regeleinheit kann den üblicherweise zeitlich aufgelösten Messwert getaktet und/oder wiederholt empfangen und/oder mit dem Schwellwert vergleichen. Der Schwellwert ist üblicherweise konstant. Grundsätzlich ist es denkbar, dass der Schwellwert nicht konstant, sondern veränderbar ist, insbesondere in Abhängigkeit von zukünftig geplanten Betriebszeiten und/oder Betriebsparametern des Multiröhren-Röntgenstrahlergehäuses.

Der Betrieb des Multiröhren-Röntgenstrahlergehäuses hängt typischerweise von der Einhaltung regulatorischer Normen und/oder Vorgaben des Gesetzgebers und/oder des Herstellers des Multiröhren-Röntgenstrahlergehäuses ab. Solche Normen und/oder Vorgaben betreffen insbesondere einen sicheren Betrieb des Röntgenstrahlergehäuses, um die Gefährdung für einen Nutzer des Multiröhren-Röntgenstrahlergehäuses und/oder für einen Patienten zu minimieren. Diesbezüglich kommen insbesondere allgemein bekannte Normen zur Isolation von Hochspannung führenden Bauteilen zur Anwendung. Dieser Aspekt betrifft somit insbesondere das erfindungsgemäße Merkmal, wonach der erste Gehäuseabschnitt elektrisch leitfähig ausgebildet ist. Wie zuvor ausgeführt hängt dessen Leitfähigkeit unter anderem von dessen Temperatur ab, welche sich im Betrieb in Abhängigkeit der Betriebsparameter erhöhen kann.

Das Vergleichsergebnis gibt insbesondere an, inwiefern der Betrieb des Multiröhren-Röntgenstrahlergehäuses fortgeführt werden kann oder ob die Abschaltung zu erfolgen hat. Es ist denkbar, dass das Vergleichsergebnis angibt, dass die Abschaltung aufrechterhalten wird. Das Vergleichsergebnis kann zusätzlich typischerweise angeben, dass die Hochspannung angeschaltet wird. In diesem Fall ist die Schalteinrichtung zur Anschaltung der Hochspannung in Abhängigkeit des Vergleichsergebnisses ausgebildet. Das Vergleichsergebnis ist insbesondere binär und/oder üblicherweise zeitlich variabel.

Die Schalteinrichtung kann zur Abschaltung und/oder zur Anschaltung der Hochspannung mit der Hochspannungsversorgung verbunden sein. Alternativ oder zusätzlich kann die Schalteinrichtung einen oder mehrere Schalter umfassen, welche nach der Abschaltung der Hochspannung das Führen der Hochspannung in der einzelnen Hochspannungszuführung unterbrechen und/oder welche nach der Anschaltung der Hochspannung das Führen der Hochspannung in der einzelnen Hochspannungszuführung ermöglichen. Eine weitere Alternative betrifft die Möglichkeit, dass der Hochspannungserzeuger bei der Abschaltung abgeschaltet und/oder bei der Anschaltung angeschaltet wird.

Eine Ausführungsform sieht vor, dass die Kühleinrichtung eine Kühlvorrichtung aufweist, welche zur Temperierung des ersten elektrisch leitfähigen Gehäuseabschnitts oberhalb eines unteren Temperaturschwellwerts und/oder unterhalb eines oberen Temperaturschwellwerts ausgebildet ist. Durch die Temperierung des ersten elektrisch leitfähigen Gehäuseabschnitts wird insbesondere die temperaturabhängige elektrische Leitfähigkeit geregelt. Das Regeln der temperaturabhängigen elektrischen Leitfähigkeit umfasst insbesondere ein Stabilisieren und/oder ein Limitieren und/oder ein Etablieren des über den ersten elektrisch leitfähigen Gehäuseabschnitt abfallenden Stroms. Die Temperierung des ersten elektrisch leitfähigen Gehäuseabschnitts ermöglicht vorteilhafterweise, dass der im Wesentlichen lineare Potentialverlauf entlang des ersten elektrisch leitfähigen Gehäuseabschnitts im Betrieb des Multiröhren-Röntgenstrahlergehäuses aufrechterhalten wird. Die Kühlvorrichtung kann aktiv oder passiv ausgestaltet sein. Ein Beispiel der passiven Ausgestaltung ist z.B. eine entsprechende Dimensionierung oder Form der Oberfläche des Gehäuses, insbesondere einer Seitenfläche. Die Form der Oberfläche kann die die Oberfläche vergrößernde Struktur umfassen. Ein Beispiel der aktiven Ausgestaltung ist, dass die Kühlvorrichtung einen Fluid-Kühlmedium-Wärmetauscher aufweist und/oder eine erzwungene Konvektion bewirken kann. Die Temperierung des Kühlmediums umfasst in diesem Fall insbesondere ein Einstellen, vorzugsweise ein Erhöhen und/oder ein Verringern, der Temperatur des Kühlmediums. Die aktive Kühlvorrichtung stellt insbesondere die Temperatur des Kühlmediums derart ein, dass die Temperatur am ersten elektrisch leitfähigen Gehäuseabschnitt oberhalb des unteren Temperaturschwellwerts und/oder unterhalb des oberen Temperaturschwellwerts ist. Der untere Temperaturschwellwert und der obere Temperaturschwellwert bilden insbesondere ein Temperaturintervall, in welchem die Betriebstemperatur des ersten elektrisch leitfähigen Gehäuseabschnitts vorzugsweise liegt. Der obere Temperaturschwellwert und/oder der untere Temperaturschwellwert kann insbesondere in der Speichereinheit abgespeichert sein. Die Kühlvorrichtung kann vorzugsweise mittels einer Schnittstelle auf den abgespeicherten oberen Temperaturschwellwert und/oder unteren Temperaturschwellwert zugreifen.

Eine Ausführungsform sieht vor, dass die Kühlvorrichtung zur Temperierung desjenigen Kühlmediums ausgebildet ist, welches sich innerhalb und/oder außerhalb des Gehäuses befindet und mit dem ersten elektrisch leitfähigen Gehäuseabschnitt direkt wechselwirkt. Diese Ausführungsform ist insbesondere vorteilhaft, weil dabei die Temperierung näher oder unmittelbar am ersten elektrisch leitfähigen Gehäuseabschnitt und somit vorzugsweise schneller und/oder präziser geschieht.

Eine Ausführungsform sieht vor, dass das Multiröhren-Röntgenstrahlergehäuse ferner einen Temperatursensor zur Messung eines die elektrische Leitfähigkeit des ersten elektrisch leitfähigen Gehäuseabschnitts abbildenden Temperaturwerts als Messwert aufweist. Insbesondere die Kühlvorrichtung ist zur Temperierung des Kühlmediums in Abhängigkeit des Temperaturwerts ausgebildet ist. Diese Ausführungsform ermöglicht insbesondere ein direktes Regeln der Temperatur des Kühlmediums.

Eine Ausführungsform sieht vor, dass das Multiröhren-Röntgenstrahlergehäuse ferner einen Stromsensor zur Messung eines über den ersten leitfähigen Gehäuseabschnitt abfließenden Stromwerts als Messwert aufweist. Die Messung kann insbesondere dadurch erfolgen, dass ein Strom zwischen der Kathode und der Anode und/oder ein Strom zwischen der Anode zu Masse und/oder ein Strom zwischen der Kathode zu Masse gemessen wird, welche regelmäßig die Summe über alle Strompfade abbilden. Vorzugsweise kann davon der Stromwert abgeleitet oder ermittelt werden. Insbesondere die Kühlvorrichtung ist zur Temperierung des Kühlmediums in Abhängigkeit des Stromwerts ausgebildet ist. Der Stromwert bildet insbesondere den über den ersten leitfähigen Gehäuseabschnitt abfließenden Strom ab. Bei dieser Ausführungsform kann der obere Temperaturschwellwert und/oder der untere Temperaturschwellwert einem oberen Stromschwellwert bzw. einem unteren Stromschwellwert zugeordnet sein oder im Wesentlichen entsprechen. Die Zuordnung der Schwellwerte kann in einer Zuordnungstabelle erfolgt sein, welche beispielsweise in der Speichereinheit abgespeichert ist.

Eine Ausführungsform sieht vor, dass der erste elektrisch leitfähige Gehäuseabschnitt Flintglas, Proceram, Siliciumnitrid, Siliciumcarbid, Zirkonoxid, Silizium und/oder ein dotiertes Material aufweist. Es ist grundsätzlich denkbar, dass der erste elektrisch leitfähige Gehäuseabschnitt ausschließlich eines der vorgenannten elektrisch leitfähigen Materialien oder eine Kombination der vorgenannten elektrisch leitfähigen Materialien in einer Materialzusammensetzung aufweist. Die Materialzusammensetzung kann verschiedene Schichten mit einem oder mehreren der vorgenannten elektrisch leitfähigen Materialien umfassen. Es ist denkbar, dass eine der Schichten als Trägerschicht und/oder eine andere Schicht als Beschichtung ausgebildet ist. Insbesondere die Beschichtung kann eine der vorgenannten elektrisch leitfähigen Materialien umfassen. Die Materialzusammensetzung kann grundsätzlich weitere Materialien, typischerweise isolierende Materialien wie Glas, Kunststoff, etc. umfassen, welche beispielsweise als Trägerschicht eingesetzt sind. Die Materialzusammensetzung kann alternativ zum Schichtaufbau in einer Mischform z.B. aus einem verfestigten Pulver vorliegen.

Eine Ausführungsform sieht vor, dass das Multiröhren-Röntgenstrahlergehäuse eine erste Buchse zur Aufnahme eines ersten Hochspannungskontakts aufweist, wobei die erste Buchse ein Befestigungsmittel, insbesondere eine Klemmmittel, ein Schraubmittel und/oder ein Steckmittel, aufweist zum lösbaren Koppeln einer ersten Hochspannungselektrode mit dem ersten Hochspannungskontakt. Diese Ausführungsform ermöglicht vorteilhafterweise ein einfaches Austauschen der ersten Hochspannungselektrode oder des die erste Hochspannungselektrode enthaltenden Bauteils, beispielsweise einer Röntgenröhre. Das lösbare Koppeln umfasst insbesondere das Ankoppeln und/oder Abkoppeln. Grundsätzlich ist es denkbar, dass in dem Gehäuse zusätzlich eine zweite Buchse oder weitere Buchsen angeordnet sind, welche im Wesentlichen baugleich ausgestaltet sein können. Weiterhin ist es denkbar, dass für jeden Hochspannungskontakt jeweils eine Hochspannungselektrode in je einer individuellen Buchse lösbar koppelbar ist.

Eine Ausführungsform sieht vor, dass in der ersten Buchse eine isolierende Tülle zur elektrischen Isolation der koppelbaren Hochspannungselektrode von der ersten Buchse angeordnet ist. Bei dieser Ausführungsform ist insbesondere die elektrische Kontaktierung zwischen der ersten Hochspannungselektrode mit dem ersten Hochspannungskontakt verbessert. Grundsätzlich können alle Buchsen derart aufgebaut sein

Eine Ausführungsform sieht vor, dass die erste Buchse aus einem Material mit einer temperaturabhängigen elektrischen Leitfähigkeit besteht. Bei dieser Ausführungsform kann insbesondere die Hochspannungsisolation zwischen der ersten Hochspannungselektrode und dem ersten Hochspannungskontakt vereinfacht werden, weil sich entlang der ersten Buchse vorteilhafterweise ein linearer Potentialverlauf einstellt, welcher die im Zusammenhang mit dem ersten elektrisch leitfähigen Gehäuseabschnitt beschriebenen Vorteile teilt. Die erste Buchse kann somit aus demselben Material und/oder derselben Materialzusammensetzung wie der erste elektrisch leitfähige Gehäuseabschnitt aufgebaut sein. Grundsätzlich können alle Buchsen derart aufgebaut sein.

Eine Ausführungsform sieht vor, dass zwischen der ersten Buchse und dem Gehäuse eine Dichtungseinrichtung zur Abdichtung des Gehäuses angeordnet ist. Bei dieser Ausführungsform ist insbesondere die Buchse ein Teil des Gehäuses und in eine Seitenfläche des Gehäuses integriert. Es ist grundsätzlich denkbar, dass die Buchse in die erste der zumindest einen Seitenfläche mit dem ersten elektrisch leitfähigen Gehäuseabschnitt integriert ist. Integriert bedeutet insbesondere, dass das Gehäuse eine Ausnehmung aufweist, welche die Befestigung der ersten Buchse an dem Gehäuse und das Durchdringen des Gehäuses mit der ersten Buchse ermöglicht. Die Abdichtung verhindert insbesondere ein Austreten des Kühlmediums durch den technisch bedingten Spalt zwischen der ersten Buchse und dem Gehäuse. Grundsätzlich kann für jede derartige Buchse eine individuelle Dichtungseinrichtung zur Abdichtung des Gehäuses angeordnet sein.

Eine erfindungsgemäße Multiröhren-Röntgenstrahlenquelle weist
- zumindest ein Multiröhren-Röntgenstrahlergehäuse und
- mehrere Röntgenröhren auf,
wobei jede Röntgenröhre ein Vakuumgehäuse mit einer Kathode und einer Anode zur Generierung von Röntgenstrahlen umfasst, wobei zwischen der Kathode und der Anode eine Beschleunigungsstrecke für emittierte Elektronen vorgesehen ist und wobei die Anoden oder die Kathoden die Hochspannungselektroden bilden und jeweils mit den Hochspannungskontakten gekoppelt sind.

Die erfindungsgemäße Multiröhren-Röntgenstrahlenquelle weist das zumindest eine Multiröhren-Röntgenstrahlergehäuse auf und teilt somit die zuvor beschriebenen Vorteile und Ausgestaltung desselben.

Die Multiröhren-Röntgenstrahlenquelle kann vorteilhafterweise aus mehreren Multiröhren-Röntgenstrahlergehäusen zusammengesetzt sein, in welche die Röntgenröhren eingesetzt sind. Der Abstand zwischen den jeweiligen Multiröhren-Röntgenstrahlergehäusen kann vorteilhafterweise minimiert bzw. null sein, weil durch den ersten elektrisch leitfähigen Gehäuseabschnitt die Isolation gegen die Hochspannung innerhalb der jeweiligen Multiröhren-Röntgenstrahlergehäusen ermöglicht ist, insbesondere wenn wenigstens ein einzelner solcher Gehäuseabschnitt eines der Multiröhren-Röntgenstrahlergehäuse räumlich zwischen benachbarten Multiröhren-Röntgenstrahlergehäusen angeordnet ist. Vorteilhafterweise sind die jeweiligen elektrisch leitfähigen Gehäuseabschnitt nebeneinander angeordnet. Die erfindungsgemäße Multiröhren-Röntgenstrahlenquelle kann somit vorteilhafterweise derart aufgebaut sein, dass zwischen wenigstens zwei Röntgenröhren, welche jeweils in verschiedenen benachbarten Multiröhren-Röntgenstrahlergehäusen eingesetzt sind, derselbe Abstand ist, wie zwischen benachbarten Röntgenröhren innerhalb eines Multiröhren-Röntgenstrahlergehäuses. In anderen Worten ist der Abstand benachbarter Röntgenröhren und somit typischerweise der Brennfleck auf der Anode äquidistant, unabhängig davon, ob die benachbarten Röntgenröhren in demselben oder in verschiedenen Multiröhren-Röntgenstrahlergehäusen eingesetzt sind.

Das Vakuumgehäuse einer der Röntgenröhren kann zumindest teilweise von dem elektrisch isolierenden Kühlmedium umgeben sein. Zwischen einem Abschnitt des Vakuumgehäuses und dem ersten elektrisch leitfähigen Gehäuseabschnitt kann sich elektrisch isolierendes Kühlmedium befinden. Der erste elektrisch leitfähige Gehäuseabschnitt ist vorzugsweise im Wesentlichen parallel zur nahe gelegensten Beschleunigungsstrecke bzw. zu den Beschleunigungsstrecken angeordnet ist. In anderen Worten sind die zum ersten elektrisch leitfähigen Gehäuseabschnitt nahe gelegensten Röntgenröhren typischerweise parallel oder im Wesentlichen parallel oder in einem spitzen Winkel zu diesem Gehäuseabschnitt ausgerichtet. In diesem Fall weist der erste elektrisch leitfähige Gehäuseabschnitt vorzugsweise eine Ausdehnung parallel zur Beschleunigungsstrecke auf, welche mindestens der Länge der nahe gelegensten Beschleunigungsstrecke entspricht.

Die mehreren Röntgenröhren können grundsätzlich parallel oder gefächert ausgerichtet sein. Diese Ausrichtung bezieht sich insbesondere auf den Zentralstrahl der jeweils generierten Röntgenstrahlen. In anderen Worten können die Vakuumgehäuse mit den Beschleunigungsstrecken der mehreren Röntgenröhren grundsätzlich parallel ausgerichtet sein und gleichzeitig bei einer Emission der Röntgenstrahlen senkrecht zu den Beschleunigungsstrecken durch die Verdrehung einzelner Röntgenröhren die Röntgenstrahlen gefächert ausgerichtet sein.

Typischerweise ist jede Röntgenröhre allein betrachtet für die bildgebende Untersuchung eines Patienten ausgebildet. Alternativ kann eine Röntgenröhre für eine Werkstoffprüfung vorgesehen sein. Die bildgebende Untersuchung kann insbesondere eine Angiographie, Computertomographie, Mammographie oder Radiographie sein.

Die emittierten Röntgenstrahlen sind typischerweise auf einen Untersuchungsbereich, beispielsweite mit dem Patienten oder dem Werkstoff, ausgerichtet. Röntgenstrahlen einer einzelnen Röntgenröhre decken üblicherweise einen Teil des Untersuchungsbereichs ab, welcher bis zu einschließlich 100% betragen kann. Alternativ kann die einzelne Röntgenröhre keine 100%, insbesondere weniger als 100% des Untersuchungsbereichs abdecken. Vorzugsweise decken die Röntgenstrahlen aller oder zumindest eines Teils der Röntgenröhren den Untersuchungsbereich zu 100%, sprich ganz ab.

Die mehreren Röntgenröhren sind typischerweise derart ansteuerbar, dass sie gleichzeitig oder konsekutiv Röntgenstrahlen emittieren können. Eine Abfolge der Röntgenstrahlenemission kann umfassen, dass zwei benachbarte Röntgenröhren Röntgenstrahlen gleichzeitig während einer Übergangszeit emittieren, welche kürzer ist als die Zeit der Röntgenstrahlenemission je Röntgenröhre. Ansteuerverfahren der mehreren Röntgenröhren ermöglichen insbesondere eine bildgebende Untersuchung, beispielsweise umfassend eine zweidimensionale Durchleuchtung-, eine Tomosynthese- oder eine projektionsbasierte dreidimensionale Volumenrekonstruktion.

Die mehreren Röntgenröhren sind vorteilhafterweise baugleich. Aufgrund der höheren Stückzahlen kann typischerweise ein Preisvorteil vorhanden sein. Zusätzlich ermöglicht die Baugleichheit, dass weniger unterschiedliche Komponenten in dem Multiröhren-Röntgenstrahlergehäuse verbaut sind, was unter anderem eine Wartung vereinfachen kann. Alternativ kann grundsätzlich eine Röntgenröhre sich von einer anderen Röntgenröhre insbesondere in Hinblick auf die Ausgestaltung der Anode und/oder Kathode unterscheiden.

Im Folgenden ist eine mögliche Ausgestaltung einer Röntgenröhre beschrieben, welche grundsätzlich für die anderen Röntgenröhren gelten kann:
Die Kathode umfasst typischerweise einen Elektronenemitter. Der Elektronenemitter ist zum Generieren eines Brennflecks auf der Anode mittels Elektronen ausgebildet. Der Elektronenemitter kann einen Feldeffekt-Emitter oder einen thermionischen Emitter aufweisen. Der thermionische Emitter kann ein Wendelemitter oder Flachemitter sein.

Die Elektronenemission beim Feldeffekt-Emitter wird typischerweise durch das Anlegen einer Gatespannung erwirkt, welche durch das in den Spitzen der Nanoröhrchen auftretenden elektrischen Felds die Elektronen aus diesen Nanoröhrchen extrahiert, wodurch der Elektronenstrom gebildet wird. Zusätzlich zum Schalten mittels der Gatespannung kann das Sperren eines generierten Elektronenstroms mittels eines Sperrgitters erfolgen. Der Feldeffekt-Emitter weist typischerweise eine Vielzahl an Nanoröhrchen, beispielsweise aus Kohlenstoff oder Silizium oder Molybdän, auf.

Die emittierten Elektronen werden von dem Elektronenemitter in Richtung der Anode entlang der Beschleunigungsstrecke beschleunigt und generieren bei der Wechselwirkung in dem Brennfleck die Röntgenstrahlung. Die generierte Röntgenstrahlung weist üblicherweise eine maximale Energie von bis zu 150 keV in Abhängigkeit von der zwischen dem Elektronenemitter und der Anode angelegten Beschleunigungsspannung auf. Die Beschleunigungsspannung entspricht bei einer unipolaren Röntgenröhre typischerweise der Hochspannung und bei einer bipolaren Röntgenröhre regelmäßig dem doppelten Betrag der Hochspannung.

Die Anode kann als Drehanode oder Stehanode ausgebildet sein. Für die vorliegende Erfindung können Stehanoden bevorzugt sein, welche typischerweise kleinere Gehäuse und einen günstigeren Aufbau umfassen. Eine womöglich geringere Röntgenstrahlendosis einer Stehanode im Vergleich zu einer Drehanode kann beispielsweise durch die Erhöhung der Anzahl an Anode kompensiert werden.

Eine Ausführungsform sieht vor, dass die mehreren Röntgenröhren vollständig innerhalb des Gehäuses angeordnet sind. In diesem Fall weist das Gehäuse typischerweise das Röntgenstrahlenaustrittfenster auf, welches typischerweise nahe bei der Anode der jeweiligen Röntgenröhren angeordnet ist. Das Röntgenstrahlenaustrittfenster kann in mehrere Teilfenster unterteilt sein.

Eine zur vorherigen Ausführungsform alternative Ausführungsform sieht vor, dass zumindest eine der Röntgenröhren aus dem Gehäuse derart hinausragt, dass an der Anode generierte Röntgenstrahlen von dem Gehäuse weg propagieren. Dass die generierten Röntgenstrahlen von dem Gehäuse weg propagieren, bedeutet insbesondere, dass die Röntgenstrahlen außerhalb des Gehäuses ohne Abschwächung an einer Seitenfläche des Gehäuses generiert werden können. Die Röntgenstrahlen, zumindest deren Zentralstrahl, können zur Gehäuseoberfläche insbesondere parallel oder senkrecht oder in einem Winkel zwischen 0 und 90° ausgerichtet sein. Besonders vorteilhafterweise ragen alle Röntgenröhren derart aus dem Gehäuse raus, dass an den Anoden generierte Röntgenstrahlen von dem Gehäuse weg propagieren.

Bei dieser Ausführungsform kann vorteilhafterweise eine Röntgenröhre vergleichsweise einfach gewechselt werden, insbesondere wenn das Multiröhren-Röntgenstrahlergehäuse die erste Buchse zur Aufnahme des ersten Hochspannungskontakts aufweist, wobei die erste Buchse das Klemmmittel, das Schraubmittel und/oder das Steckmittel aufweist zum lösbaren Koppeln der ersten Hochspannungselektrode mit dem ersten Hochspannungskontakt. In diesem Fall kann die gekoppelte Röntgenröhre gelöst werden, beispielsweise für eine Wartung und/oder einen Austausch.

Eine Ausführungsform sieht vor, dass ein Abstand zwischen dem ersten elektrisch leitfähigen Gehäuseabschnitt und einer der Röntgenröhren geringer ist als eine dazwischen liegende Isolationsstrecke entlang elektrisch isolierenden Kühlmediums. Diese Ausführungsform ist insbesondere dadurch möglich, dass sich aufgrund des ersten elektrisch leitfähigen Gehäuseabschnitts der lineare Potentialverlauf einstellt und somit der sichere Betrieb derjenigen Röntgenröhre sichergestellt ist.

Eine erfindungsgemäße Röntgen-Einrichtung weist
- die Multiröhren-Röntgenstrahlenquelle und
- einen Röntgendetektor.

Die erfindungsgemäße Röntgen-Einrichtung weist das zumindest eine Multiröhren-Röntgenstrahlergehäuse auf und teilt somit die zuvor beschriebenen Vorteile und Ausgestaltung desselben.

Der Röntgendetektor ist zu einem Detektieren derjenigen Röntgenstrahlung ausgebildet, welche durch den Untersuchungsbereich hindurchpropagiert. Beim Detektieren wird insbesondere ein Schwächungsprofil erfasst. Die detektierte Röntgenstrahlung kann in einem Rekonstruktionsrechner beispielsweise gemäß der bildgebenden Untersuchung verwendet werden, um ein 2D- oder 3D-Bild zu rekonstruieren. Grundsätzlich sind auch Bildserien über die Zeit rekonstruierbar.

Eine erfindungsgemäße Computertomographie-Einrichtung, aufweisend
- mehrere Röntgen-Einrichtungen, wobei die Gehäuse der Multiröhren-Röntgenstrahlergehäuse Kreisbogen-artig ausgebildet sind, wobei die Kreisbogen-artigen Gehäuse mindestens 180° abdecken und wobei ein Abstand zwischen benachbarten Röntgenröhren äquidistant ist.

Die erfindungsgemäße Computertomographie-Einrichtung weist das zumindest eine Multiröhren-Röntgenstrahlergehäuse auf und teilt somit die zuvor beschriebenen Vorteile und Ausgestaltung desselben.

In anderen Worten sind die mehreren Röntgeneinrichtungen insbesondere über den ersten elektrisch leitfähigen Gehäuseabschnitt miteinander verbunden bzw. stehen darüber in Kontakt. Bei einer Kreisbogen-artigen Ausgestaltung ist eine Flächennormale des ersten elektrisch leitfähigen Gehäuseabschnitts im Wesentlichen tangential ausgerichtet ist. Vorteilhafterweise sind die mehreren Röntgenröhren, insbesondere deren Röntgenstrahlen, gefächert ausgerichtet.

Bei der Beschreibung der Vorrichtung erwähnte Merkmale, Vorteile oder alternative Ausführungsformen sind ebenso auf ein Verfahren zu übertragen und umgekehrt. Mit anderen Worten können Ansprüche auf das Verfahren mit Merkmalen der Vorrichtung weitergebildet sein. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Multiröhren-Röntgenstrahlergehäuse,
Fig. 2 ein erstes Ausführungsbeispiel des Multiröhren-Röntgenstrahlergehäuses,
Fig. 3 ein zweites Ausführungsbeispiel des Multiröhren-Röntgenstrahlergehäuses,
Fig. 4 ein drittes Ausführungsbeispiel des Multiröhren-Röntgenstrahlergehäuses,
Fig. 5 und Fig. 6 ein viertes Ausführungsbeispiel des Multiröhren-Röntgenstrahlergehäuses,
Fig. 7 eine Multiröhren-Röntgenstrahlenquelle,
Fig. 8 ein weiteres Ausführungsbeispiel der Multiröhren-Röntgenstrahlenquelle und
Fig. 9 eine Röntgen-Einrichtung.

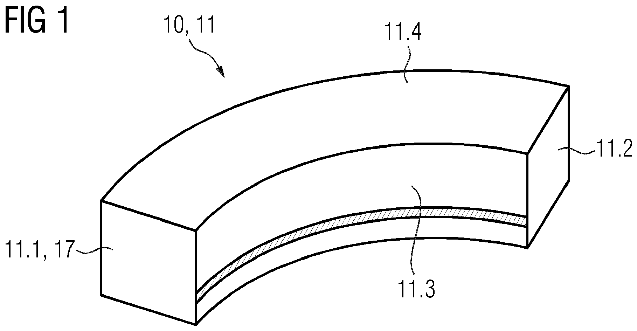

Fig. 1 zeigt eine perspektivische Ansicht eines Multiröhren-Röntgenstrahlergehäuses 10.

Das Multiröhren-Röntgenstrahlergehäuse 10 weist ein Gehäuse 11 mit zumindest einer Seitenfläche 11.1 ... 11.N auf. Innerhalb des Gehäuses 11 weist das Multiröhren-Röntgenstrahlergehäuse 10 eine Hochspannungsversorgung 12 und eine Kühleinrichtung mit einem elektrisch isolierenden Kühlmedium 13 auf, welcher von den Seitenflächen 11.1 ... 11.N in Fig. 1 verdeckt sind.

Das Gehäuse 11 ist in Fig. 1 Kreisbogen-artig als länglicher Stab gekrümmt ausgebildet. Eine Flächennormale des ersten elektrisch leitfähigen Gehäuseabschnitts 17 ist somit im Wesentlichen tangential ausgerichtet. Alternativ kann das Gehäuse 11 ungekrümmt, insbesondere gerade ausgestaltet sein.

Die Hochspannungsversorgung 12 weist innerhalb des Gehäuses 11 eine einzelne Hochspannungszuführung 14 zur Führung einer Hochspannung und mehrere an der einzelnen Hochspannungszuführung 14 parallel geschaltete Hochspannungskontakte 15.1 ... 15.N auf. Die Hochspannungskontakte 15.1 ... 15.N sind jeweils mit einer Hochspannungselektrode 16.1 ... 16.N zur Bereitstellung der Hochspannung koppelbar und liegen auf demselben Hochspannungspotential.

Eine erste der zumindest einen Seitenflächen 11.1 ... 11.N weist einen ersten elektrisch leitfähigen Gehäuseabschnitt 17 mit einer temperaturabhängigen elektrischen Leitfähigkeit auf. In dem in Fig. 1 gezeigten Ausführungsbeispiel ist die mit 11.1 gekennzeichnete Seitenfläche derart ausgestaltet. Eine weitere bevorzugte Seitenfläche ist die mit 11.2 gekennzeichnete Seitenfläche. Beispielsweise könnte diese oder auch beide Seitenflächen 11.1, 11.2 einen ersten 17 bzw. einen zweiten elektrisch leitfähigen Gehäuseabschnitt aufweisen. Selbstverständlich könnten auch die anderen Seitenflächen 11.3, 11.4 oder die abgewandten Seitenflächen 11.N einen elektrisch leitfähigen Gehäuseabschnitt aufweisen. Zwischen dem ersten elektrisch leitfähigen Gehäuseabschnitt 17 und einem der Hochspannungskontakte 15.1 ... 15.N befindet sich elektrisch isolierendes Kühlmedium 13. Die elektrische Leitfähigkeit bei einem unteren Temperaturschwellwert beträgt vorzugsweise mindestens 10^-8 S/m und/oder bei einem oberen Temperaturschwellwert maximal 10^-4 S/m. Der erste elektrisch leitfähige Gehäuseabschnitt 17 weist insbesondere Flintglas, Proceram, Siliciumnitrid, Siliciumcarbid, Zirkonoxid, Silizium und/oder ein dotiertes Material auf.

Das Multiröhren-Röntgenstrahlergehäuse 10 weist ferner eine Regeleinheit 18 und eine Schalteinrichtung 20 auf, welche in Fig. 1 nicht gezeigt sind. Die Regeleinheit 18 weist eine Schnittstelle 19 zum Empfang eines die elektrische Leitfähigkeit des ersten elektrisch leitfähigen Gehäuseabschnitts 17 abbildenden Messwerts auf und ist zum Vergleichen des Messwerts mit einem Schwellwert ausgebildet. Die Schalteinrichtung 20 ist zur Abschaltung der Hochspannung in Abhängigkeit des Vergleichsergebnisses ausgebildet.

In Fig. 1 ist ein optionales Röntgenstrahlenaustrittfenster 21 in der Seitenfläche 11.3 angedeutet, welches je nach Ausgestaltung in dieser Seitenfläche oder in einer anderen Seitenfläche des Multiröhren-Röntgenstrahlergehäuse 10 vorgesehen sein kann.

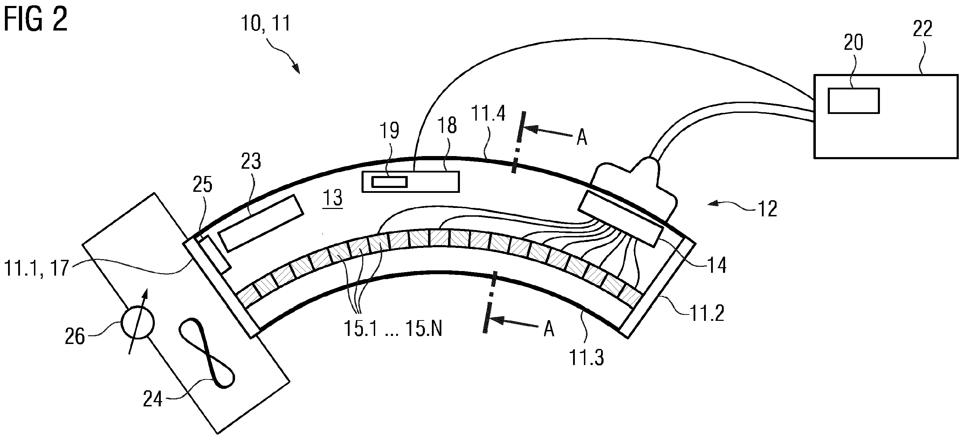

Fig. 2 zeigt ein erstes Ausführungsbeispiel des Multiröhren-Röntgenstrahlergehäuses 10 in einem Querschnitt entlang der Längsachse.

Vorteilhafterweise weist die Kühleinrichtung eine Kühlvorrichtung 24 auf, welche zur Temperierung des ersten elektrisch leitfähigen Gehäuseabschnitts 17 oberhalb eines unteren Temperaturschwellwerts und/oder unterhalb eines oberen Temperaturschwellwerts ausgebildet ist. In diesem Ausführungsbeispiel ist die Kühlvorrichtung 24 beispielhaft aktiv ausgeführt. Gezeigt ist eine Kühlvorrichtung 24, welche mittels einer erzwungenen Konvektion der Umgebungsluft außerhalb des Gehäuses 11 den ersten elektrisch leitfähigen Gehäuseabschnitt 17 aktiv kühlt. Somit ist die Kühlvorrichtung 24 zur Temperierung desjenigen Kühlmediums ausgebildet, welches sich außerhalb des Gehäuses 11 befindet und mit dem ersten elektrisch leitfähigen Gehäuseabschnitt 17 direkt wechselwirkt. In einer nicht gezeigten alternativen Ausführungsform kann die Kühlvorrichtung 24 dasjenige Kühlmedium 13 kühlen, welches sich innerhalb des Gehäuses 11 befindet.

Das Gehäuse 11 ist in Fig. 2 Kreisbogen-artig ausgebildet. Bei der Kreisbogen-artigen Ausgestaltung ist eine Flächennormale des ersten elektrisch leitfähigen Gehäuseabschnitts 17 im Wesentlichen tangential in Bezug auf einen Mittelpunkt des Kreisbogens ausgerichtet.

Die Hochspannungsversorgung 12 weist einen Hochspannungserzeuger 22 auf, welcher außerhalb des Gehäuses 11 angeordnet ist. Der Hochspannungserzeuger 22 ist über ein Kabel mit der Hochspannungszuführung 14 verbunden. Der Hochspannungserzeuger 22 umfasst eine Schalteinrichtung 20 zur Abschaltung der Hochspannung. Die Regeleinheit 18 ist zum Übertragen des Vergleichsergebnisses oder eines Abschaltsignals mit der Schalteinrichtung 20 verbunden. Die Verbindung ist in diesem Ausführungsbeispiel kabelgebunden, kann alternativ kabellos erfolgen.

Das Gehäuse 11 umfasst eine Mehrzahl an Hochspannungskontakten 15.1 ... 15.N. Die in Fig. 2 gezeigte Anzahl dient rein illustratorischen Zwecken. Die Anzahl beträgt mindestens 2. Die Hochspannungszuführung 14 überträgt die Hochspannung an die Hochspannungskontakte 15.1 ... 15.N mittels galvanischer Verbindungen, beispielsweise kabelgebunden oder mittels parallelgeschalteter Leiterbahnen. Aus Gründen der Übersichtlichkeit ist nur ein Teil dieser Verbindungen in Fig. 2 dargestellt.

Die Seitenfläche 11.1 weist den ersten elektrisch leitfähigen Gehäuseabschnitt 17 auf. Das Gehäuse 11 umfasst ein Ausdehnungsausgleichgefäß 23. Ferner umfasst das Gehäuse 11 die Regeleinheit 18, welche alternativ auch außerhalb des Gehäuses 11 angeordnet sein kann. Die Regeleinheit 18 umfasst die Schnittstelle 19.

Das Multiröhren-Röntgenstrahlergehäuse 10 weist ferner einen Temperatursensor 25 zur Messung eines die elektrische Leitfähigkeit des ersten elektrisch leitfähigen Gehäuseabschnitts 17 abbildenden Temperaturwerts als Messwert und einen Stromsensor 26 zur Messung eines über den ersten elektrisch leitfähigen Gehäuseabschnitt 17 abfließenden Stromwerts als Messwert auf. Der Temperatursensor 25 ist innerhalb des Gehäuses nahe zum ersten elektrisch leitfähigen Gehäuseabschnitt 17 angeordnet. Grundsätzlich ist es möglich den Temperatursensor 25 und/oder den Stromsensor 26 vorzusehen.

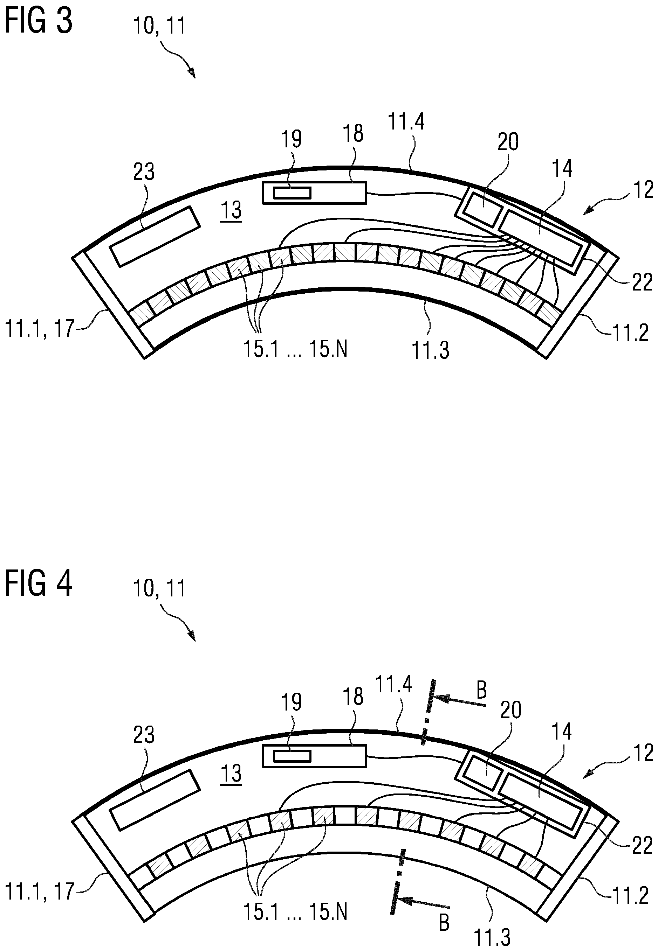

Fig. 3 zeigt ein zweites Ausführungsbeispiel des Multiröhren-Röntgenstrahlergehäuses 10 in einem Querschnitt entlang der Längsachse.

Im Vergleich zu dem in Fig. 2 gezeigten Ausführungsbeispiel ist der Hochspannungserzeuger 22 innerhalb des Gehäuses 11 angeordnet. Das Kühlmedium 13 kann insbesondere zur Kühlung des Hochspannungserzeuger 22 eingesetzt sein.

Fig. 4 zeigt ein drittes Ausführungsbeispiel des Multiröhren-Röntgenstrahlergehäuses 10 in einem Querschnitt entlang der Längsachse.

Im Wesentlichen entspricht das in Fig. 4 gezeigte Ausführungsbeispiel dem Ausführungsbeispiel der Fig. 3. Verschieden ist in erster Linie der Abstand zwischen den Hochspannungskontakten 15.1 ... 15.N, um eine Koppel- und Entkoppelbarkeit von nicht gezeigten Röntgenröhren zu verbessern.

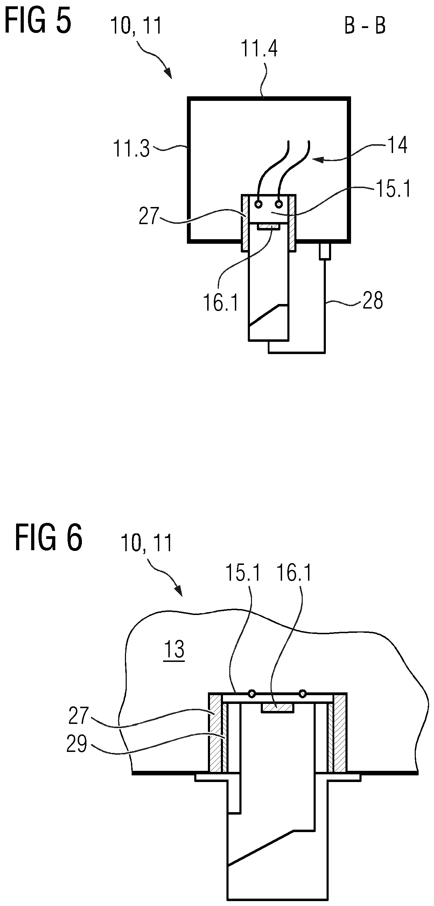

Fig. 5 zeigt ein viertes Ausführungsbeispiel des Multiröhren-Röntgenstrahlergehäuses 10 in einem Querschnitt senkrecht zur Längsachse entlang B-B, dargestellt in Fig. 4.

Das Multiröhren-Röntgenstrahlergehäuse 10 weist eine erste Buchse 27 zur Aufnahme eines ersten Hochspannungskontakts 15.1 auf. Die erste Buchse 27 ist eine Vertiefung in dem Gehäuse 11. Das Gehäuse 11 weist einen U-förmigen Querschnitt auf, wobei die erste Buchse 27 von den beiden Schenkeln eingerahmt ist. Der erste Hochspannungskontakt 15.1 ist an der Grundfläche der ersten Buchse 27 angeordnet.

Die erste Buchse 27 weist ein Befestigungsmittel 28, insbesondere ein Klemmmittel, ein Schraubmittel und/oder ein Steckmittel, auf zum lösbaren Koppeln einer ersten Hochspannungselektrode 16.1 mit dem ersten Hochspannungskontakt 15.1. Dargestellt in Fig. 5 ist eine Röntgenröhre, welche typischerweise eine Kathode und eine Anode aufweist. In diesem Ausführungsbeispiel bildet die Kathode die Hochspannungselektrode 16.1. Der Abstand zwischen dem ersten Hochspannungskontakt 15.1 und der ersten Hochspannungselektrode 16.1 dient rein illustratorischen Zwecken. Das Befestigungsmittel 28 ist als Klemmmittel, insbesondere als Spannfeder, ausgebildet.

Fig. 6 zeigt das vierte Ausführungsbeispiel des Multiröhren-Röntgenstrahlergehäuses 10 in einer vergrößerten Ansicht.

In der ersten Buchse 27 ist eine isolierende Tülle 29 zur elektrischen Isolation der koppelbaren Hochspannungselektrode 15.1 von der ersten Buchse 27 angeordnet. Die erste Buchse 27 besteht aus einem Material mit einer temperaturabhängigen elektrischen Leitfähigkeit. Die nicht auf Hochspannung liegende Hochspannungselektrode der Röntgenröhre, sprich die Anode, liegt vorzugsweise auf Massepotential.

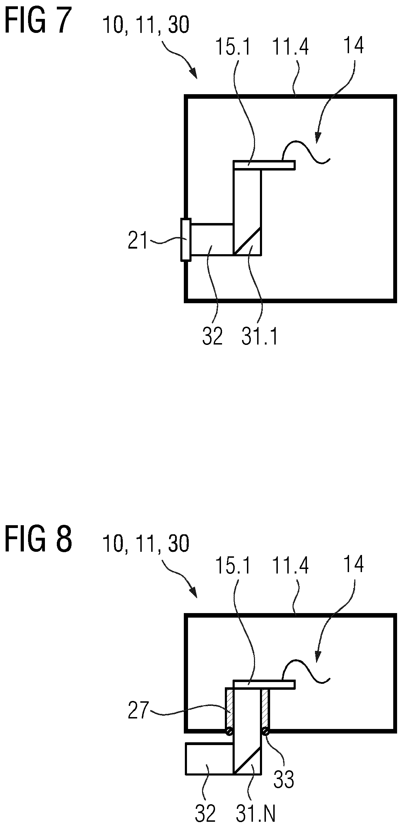

Fig. 7 zeigt eine Multiröhren-Röntgenstrahlenquelle 30 in einem Querschnitt senkrecht zur Längsachse.

Die Multiröhren-Röntgenstrahlenquelle 30 weist zumindest ein Multiröhren-Röntgenstrahlergehäuse 10 und mehrere Röntgenröhren 31.1 ... 31.N auf. Jede Röntgenröhre 31.1 ... 31.N umfasst ein Vakuumgehäuse mit einer Kathode und einer Anode zur Generierung von Röntgenstrahlen. Zwischen der Kathode und der Anode ist eine Beschleunigungsstrecke für emittierte Elektronen vorgesehen. Die Kathoden, alternativ aber nicht gezeigt die Anoden, bilden die Hochspannungselektroden 16.1 ... 16.N und sind jeweils mit den Hochspannungskontakten 15.1 ... 15.N gekoppelt. In Fig. 7 ist beispielhaft eine Röntgenröhre 31.1 in einem Querschnitt senkrecht zur Längsachse dargestellt. Die übrigen Röntgenröhren können gleichermaßen ausgestaltet sein.

Beim Ausführungsbeispiel der Fig. 7 ist die Röntgenröhre 31.1 innerhalb des Gehäuses 11 angeordnet. Zwischen der Anode und dem Röntgenstrahlenaustrittfenster 21 ist ein Kollimator 32 angeordnet. Die emittierten Röntgenstrahlen verlassen das Gehäuse 11 durch das Röntgenstrahlenaustrittfenster 21.

Fig. 8 zeigt ein weiteres Ausführungsbeispiel der Multiröhren-Röntgenstrahlenquelle 30.

Zumindest eine 31.N der Röntgenröhren ragt aus dem Gehäuse 11 derart hinaus, dass an der Anode generierte Röntgenstrahlen von dem Gehäuse 11 weg propagieren. In anderen Worten ist die Anode der Röntgenröhre 31.N außerhalb des Gehäuses 11 angeordnet. Grundsätzlich können alle Röntgenröhren 31.1 ... 31.N außerhalb des Gehäuses angeordnet sein.

Das Multiröhren-Röntgenstrahlergehäuse 10 weist die erste Buchse 27 zur Aufnahme eines Hochspannungskontakts 15.N zum lösbaren Koppeln der Röntgenröhre 31.N auf. Zwischen der ersten Buchse 27 und dem Gehäuse 11 ist eine Dichtungseinrichtung 33 zur Abdichtung des Gehäuses 11 angeordnet. Die Dichtungseinrichtung 33 kann insbesondere ringförmig oder vieleckig ausgebildet sein.

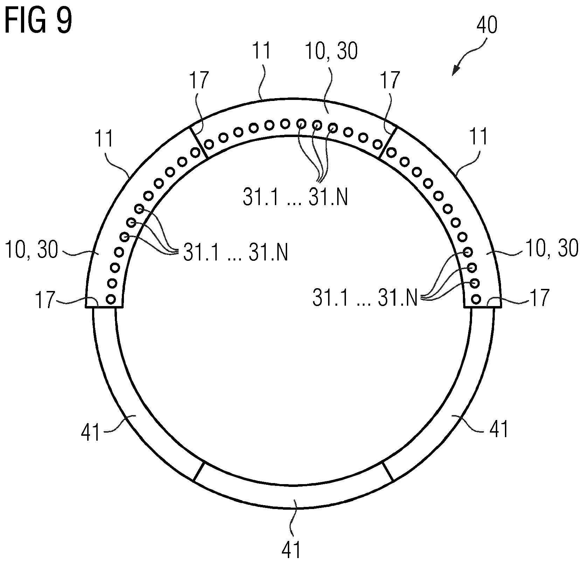

Fig. 9 zeigt eine Röntgen-Einrichtung 40 in einer Frontalansicht.

Die Röntgen-Einrichtung 40 weist die Multiröhren-Röntgenstrahlenquelle 30 und einen Röntgendetektor 41 auf. Gezeigt ist in Fig. 9 eine Computertomographie-Einrichtung, welche mehrere Röntgen-Einrichtungen 40 aufweist. Die Gehäuse 11 der Multiröhren-Röntgenstrahlergehäuse 10 sind Kreisbogen-artig ausgebildet. Die Kreisbogen-artigen Gehäuse 11 decken mindestens 180° ab. Grundsätzlich ist denkbar, dass so viele Röntgen-Einrichtungen 40 und/oder längere Kreisbogen-artige Gehäuse 11 eingesetzt werden, so dass die Kreisbogen-artigen Gehäuse 11 360° abdecken.

Der Röntgendetektor 41 deckt in Fig. 9 180° ab und ist jeweils gegenüber von den mehreren Röntgenröhren 31.1 ... 31.N angeordnet. Der Röntgendetektor 41 kann aus mehreren Einzeldetektoren bestehen oder einen zusammenhängenden Röntgendetektor bilden.

Ein Abstand zwischen den benachbarten Röntgenröhren 31.1 ... 31.N ist äquidistant. Ein Abstand zwischen dem ersten elektrisch leitfähigen Gehäuseabschnitt 17 und einer der Röntgenröhren 31.1 ... 31.N ist geringer als eine dazwischen liegende Isolationsstrecke entlang elektrisch isolierenden Kühlmediums 13.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Multiröhren-Röntgenstrahlergehäuse (10), aufweisend
- ein Gehäuse (11) mit zumindest einer Seitenfläche (11.1 ... 11.N),
- eine Hochspannungsversorgung (12) und
- eine Kühleinrichtung mit einem elektrisch isolierenden Kühlmedium (13),
wobei die Hochspannungsversorgung (12) innerhalb des Gehäuses (11) eine einzelne Hochspannungszuführung (14) zur Führung einer Hochspannung und mehrere an der einzelnen Hochspannungszuführung (14) parallel geschaltete Hochspannungskontakte (15.1 ... 15.N) aufweist,
wobei die Hochspannungskontakte (15.1 ... 15.N) jeweils mit einer Hochspannungselektrode (16.1 ... 16.N) zur Bereitstellung der Hochspannung koppelbar sind und auf demselben Hochspannungspotential liegen,
wobei eine erste der zumindest einen Seitenfläche (11.1 ... 11.N) einen ersten elektrisch leitfähigen Gehäuseabschnitt (17) mit einer temperaturabhängigen elektrischen Leitfähigkeit aufweist,
wobei sich zwischen dem ersten elektrisch leitfähigen Gehäuseabschnitt (17) und einem der Hochspannungskontakte (15.1 ... 15.N) elektrisch isolierendes Kühlmedium (13) befindet, **gekennzeichnet**
**durch** eine Regeleinheit (18), welche eine Schnittstelle (19) zum Empfang eines die elektrische Leitfähigkeit des ersten elektrisch leitfähigen Gehäuseabschnitts (17) abbildenden Messwerts aufweist und zum Vergleichen des Messwerts mit einem Schwellwert ausgebildet ist, und
**durch** eine Schalteinrichtung (20) zur Abschaltung der Hochspannung in Abhängigkeit des Vergleichsergebnisses.

2. Multiröhren-Röntgenstrahlergehäuse (10) nach Anspruch 1, wobei die Kühleinrichtung eine Kühlvorrichtung (24) aufweist, welche zur Temperierung des ersten elektrisch leitfähigen Gehäuseabschnitts (17) oberhalb eines unteren Temperaturschwellwerts und/oder unterhalb eines oberen Temperaturschwellwerts ausgebildet ist.

3. Multiröhren-Röntgenstrahlergehäuse (10) nach Anspruch 2, wobei die Kühlvorrichtung (24) zur Temperierung desjenigen Kühlmediums (13) ausgebildet ist, welches sich innerhalb und/oder außerhalb des Gehäuses befindet und mit dem ersten elektrisch leitfähigen Gehäuseabschnitt (17) direkt wechselwirkt.

4. Multiröhren-Röntgenstrahlergehäuse (10) nach einem der vorhergehenden Ansprüche,
wobei das Multiröhren-Röntgenstrahlergehäuse (10) ferner einen Temperatursensor (25) zur Messung eines die elektrische Leitfähigkeit des ersten elektrisch leitfähigen Gehäuseabschnitts (17) abbildenden Temperaturwerts als Messwert und/oder einen Stromsensor (26) zur Messung eines über den ersten elektrisch leitfähigen Gehäuseabschnitt (17) abfließenden Stromwerts als Messwert aufweist.

5. Multiröhren-Röntgenstrahlergehäuse (10) nach einem der vorhergehenden Ansprüche,
wobei die elektrische Leitfähigkeit bei einem unteren Temperaturschwellwert mindestens 10^-8 S/m und/oder bei einem oberen Temperaturschwellwert maximal 10^-4 S/m beträgt.

6. Multiröhren-Röntgenstrahlergehäuse (10) nach einem der vorhergehenden Ansprüche,
wobei der erste elektrisch leitfähige Gehäuseabschnitt (17) Flintglas, Proceram, Siliciumnitrid, Siliciumcarbid, Zirkonoxid, Silizium und/oder ein dotiertes Material aufweist.

7. Multiröhren-Röntgenstrahlergehäuse (10) nach einem der vorhergehenden Ansprüche,
wobei das Multiröhren-Röntgenstrahlergehäuse (10) eine erste Buchse (27) zur Aufnahme eines ersten Hochspannungskontakts (15.1 ... 15.N) aufweist und wobei die erste Buchse (27) ein Befestigungsmittel (28), insbesondere ein Klemmmittel, ein Schraubmittel und/oder ein Steckmittel, aufweist zum lösbaren Koppeln einer ersten Hochspannungselektrode (16.1 ... 16.N) mit dem ersten Hochspannungskontakt (15.1 ... 15.N).

8. Multiröhren-Röntgenstrahlergehäuse (10) nach Anspruch 7, wobei in der ersten Buchse (27) eine isolierende Tülle (29) zur elektrischen Isolation der koppelbaren Hochspannungselektrode (16.1 ... 16.N) von der ersten Buchse (27) angeordnet ist.

9. Multiröhren-Röntgenstrahlergehäuse (10) nach Anspruch 7 oder 8,
wobei die erste Buchse (27) aus einem Material mit einer temperaturabhängigen elektrischen Leitfähigkeit besteht.

10. Multiröhren-Röntgenstrahlergehäuse (10) nach einem der Ansprüche 7 bis 9,
wobei zwischen der ersten Buchse (27) und dem Gehäuse (11) eine Dichtungseinrichtung (33) zur Abdichtung des Gehäuses (11) angeordnet ist.

11. Multiröhren-Röntgenstrahlenquelle (30), aufweisend
- zumindest ein Multiröhren-Röntgenstrahlergehäuse (10) nach einem der vorhergehenden Ansprüche und
- mehrere Röntgenröhren (31.1 ... 31.N),
wobei jede Röntgenröhre (31.1 ... 31.N) ein Vakuumgehäuse mit einer Kathode und einer Anode zur Generierung von Röntgenstrahlen umfasst, wobei zwischen der Kathode und der Anode eine Beschleunigungsstrecke für emittierte Elektronen vorgesehen ist und
wobei die Anoden oder die Kathoden die Hochspannungselektroden (16.1 ... 16.N) bilden und jeweils mit den Hochspannungskontakten (15.1 ... 15.N) gekoppelt sind.

12. Multiröhren-Röntgenstrahlenquelle (30) nach Anspruch 11, wobei zumindest eine der Röntgenröhren (31.1 ... 31.N) aus dem Gehäuse (11) derart hinausragt, dass an der Anode generierte Röntgenstrahlen von dem Gehäuse (11) weg propagieren.

13. Multiröhren-Röntgenstrahlenquelle (30) nach einem der Ansprüche 11 oder 12,
wobei ein Abstand zwischen dem ersten elektrisch leitfähigen Gehäuseabschnitt (17) und einer der Röntgenröhren (31.1 ... 31.N) geringer ist als eine dazwischen liegende Isolationsstrecke entlang elektrisch isolierenden Kühlmediums (13).

14. Röntgen-Einrichtung (40), aufweisend
- eine Multiröhren-Röntgenstrahlenquelle (30) nach einem der Ansprüche 11 bis 13 und
- einen Röntgendetektor (41).

15. Computertomographie-Einrichtung, aufweisend
- mehrere Röntgen-Einrichtungen (40) nach Anspruch 14, wobei die Gehäuse (11) der Multiröhren-Röntgenstrahlergehäuse (10) Kreisbogen-artig ausgebildet sind, wobei die Kreisbogen-artigen Gehäuse (11) mindestens 180° abdecken und wobei ein Abstand zwischen benachbarten Röntgenröhren (31.1 ... 31.N) äquidistant ist.

## Claims

1. Multitube X-ray emitter housing (10), having
- a housing (11) with at least one side surface (11.1 ... 11.N),
- a high-voltage supply (12) and
- a cooling facility with an electrically insulating cooling medium (13),
wherein, inside the housing (11), the high-voltage supply (12) has a single high-voltage supply lead (14) for conducting a high voltage and a plurality of high-voltage contacts (15.1 ... 15.N) connected in parallel on the single high-voltage supply lead (14),
wherein the high-voltage contacts (15.1 ... 15.N) can in each case be coupled to a high-voltage electrode (16.1 ... 16.N) to provide the high voltage and are at the same high-voltage potential,
wherein a first of the at least one side surface (11.1 ... 11.N) has a first electrically conductive housing portion (17) with a temperature-dependent electrical conductivity,
wherein electrically insulating cooling medium (13) is located between the first electrically conductive housing portion (17) and one of the high-voltage contacts (15.1 ... 15.N),
**characterised**
**by** a control unit (18), which has an interface (19) for receiving a measured value representing the electrical conductivity of the first electrically conductive housing portion (17) and is embodied to compare the measured value with a threshold value and
by a switching facility (20) for switching off the high voltage in dependence on the result of the comparison.

2. Multitube X-ray emitter housing (10) according to claim 1, wherein the cooling facility has a cooling apparatus (24) embodied to stabilise the temperature of the first electrically conductive housing portion (17) above a lower temperature threshold value and/or below an upper temperature threshold value.

3. Multitube X-ray emitter housing (10) according to claim 2, wherein the cooling apparatus (24) is embodied to stabilise the temperature of the cooling medium (13) which is located inside and/or outside the housing and interacts directly with the first electrically conductive housing portion (17).

4. Multitube X-ray emitter housing (10) according to one of the preceding claims,
wherein the multitube X-ray emitter housing (10) furthermore has a temperature sensor (25) for measuring a temperature value representing the electrical conductivity of the first electrically conductive housing portion (17) as a measured value and/or a current sensor (26) for measuring a current value flowing away via the first electrically conductive housing portion (17) as a measured value.

5. Multitube X-ray emitter housing (10) according to one of the preceding claims,
wherein the electrical conductivity is at least 10^-8 S/m at a lower temperature threshold value and/or at most 10^-4 S/m at an upper temperature threshold value.

6. Multitube X-ray emitter housing (10) according to one of the preceding claims,
wherein the first electrically conductive housing portion (17) has flint glass, proceram, silicon nitride, silicon carbide, zirconia, silicon and/or a doped material.

7. Multitube X-ray emitter housing (10) according to one of the preceding claims,
wherein the multitube X-ray emitter housing (10) has a first bushing (27) for accommodating a first high-voltage contact (15.1 ... 15.N) and wherein the first bushing (27) has an attaching means (28), in particular a clamping means, a screwing means and/or a plug-in means for detachably coupling a first high-voltage electrode (16.1 ... 16.N) to the first high-voltage contact (15.1 ... 15.N).

8. Multitube X-ray emitter housing (10) according to claim 7, wherein an insulating grommet (29) is arranged in the first bushing (27) for electrically insulating the couplable high-voltage electrode (16.1 ... 16.N) from the first bushing (27).

9. Multitube X-ray emitter housing (10) according to claim 7 or 8,
wherein the first bushing (27) is made of a material with a temperature-dependent electrical conductivity.

10. Multitube X-ray emitter housing (10) according to one of claims 7 to 9,
wherein a sealing facility (33) for sealing the housing (11) is arranged between the first bushing (27) and the housing (11) .

11. Multitube X-ray source (30), having
- at least one multitube X-ray emitter housing (10) according to one of the preceding claims and
- a plurality of X-ray tubes (31.1 ... 31.N),
wherein each X-ray tube (31.1 ... 31.N) includes a vacuum housing with a cathode and an anode for generating X-rays,
wherein an acceleration path for emitted electrons is provided between the cathode and the anode and
wherein the anodes or the cathodes form the high-voltage electrodes (16.1 ... 16.N) and are in each case coupled to the high-voltage contacts (15.1 ... 15.N).

12. Multitube X-ray source (30) according to claim 11,
wherein at least one of the X-ray tubes (31.1 ... 31.N) protrudes from the housing (11) such that X-rays generated at the anode propagate away from the housing (11).

13. Multitube X-ray source (30) according to one of claims 11 or 12,
wherein a distance between the first electrically conductive housing portion (17) and one of the X-ray tubes (31.1 ... 31.N) is less than an intermediate insulation distance along electrically insulating cooling medium (13).

14. X-ray facility (40), having
- a multitube X-ray source (30) according to one of claims 11 to 13 and
- an X-ray detector (41).

15. Computed tomography facility, having
- a plurality of X-ray facilities (40) according to claim 14, wherein the housings (11) of the multitube X-ray emitter housings (10) are embodied as arc-shaped, wherein the arc-shaped housings (11) cover at least 180° and wherein a distance between adjacent X-ray tubes (31.1 ... 31.N) is equidistant.

## Revendications

1. Boîtier (10) d'émetteur de rayons X à plusieurs tubes, comportant
- un boîtier (11) ayant au moins une surface (11.1 ... 11.N) latérale,
- une alimentation (12) en haute tension et
- un dispositif de refroidissement ayant un milieu (13) de refroidissement isolant du point de vue électrique,
dans lequel l'alimentation (12) en haute tension a, à l'intérieur du boîtier (11), une seule arrivée (14) de haute tension pour l'application d'une haute tension et plusieurs contacts (15.1 ... 15.N) de haute tension montés en parallèle avec la seule arrivée (14) de haute tension,
dans lequel les contacts (15.1 ... 15.N) de haute tension peuvent, pour disposer de la haute tension, être connectés à respectivement une électrode (16.1 ... 16.N) de haute tension et sont au même potentiel de haute tension,
dans lequel une première de la au moins une surface (11.1 ... 11.N) latérale a une première partie (17) de boîtier conductrice de l'électricité ayant une conductivité électrique, qui dépend de la température,
dans lequel un milieu (13) de refroidissement isolant du point de vue électrique se trouve entre la première partie (17) du boîtier conductrice de l'électricité et l'un des contacts (15.1 ... 15.N) de haute tension,
**caractérisé**
**par** une unité (18) de régulation, qui a une interface (19) de réception d'une valeur de mesure représentant la conductivité électrique de la première partie (17) du boîtier conductrice de l'électricité et qui est constituée pour comparer la valeur de mesure à une valeur de seuil, et
par un dispositif (20) de coupure pour interrompre la haute tension en fonction du résultat de la comparaison.

2. Boîtier (10) d'émetteur de rayons X à plusieurs tubes suivant la revendication 1,
dans lequel le dispositif de refroidissement a un système (24) de refroidissement, qui est constitué pour la mise en température de la première partie (17) du boîtier conductrice de l'électricité au-dessus d'une valeur de seuil inférieure de température et/ou en-dessous d'une valeur de seuil supérieure de température.

3. Boîtier (10) d'émetteur de rayons X à plusieurs tubes suivant la revendication 2,
dans lequel le système (24) de refroidissement est constitué pour la mise en température du milieu (13) de refroidissement, qui se trouve à l'intérieur et/ou à l'extérieur du boîtier et interagit directement avec la première partie (17) du boîtier conductrice de l'électricité.

4. Boîtier (10) d'émetteur de rayons X à plusieurs tubes suivant l'une des revendications précédentes,
dans lequel le boîtier (10) d'émetteur de rayons X à plusieurs tubes a en outre une sonde (25) de température pour la mesure, comme valeur de mesure, d'une valeur de température représentant la conductivité électrique de la première partie (17) du boîtier conductrice de l'électricité et/ou un capteur (26) de courant pour la mesure, comme valeur de mesure, d'une valeur du courant passant par la première partie (17) de boîtier conductrice de l'électricité.

5. Boîtier (10) d'émetteur de rayons X à plusieurs tubes suivant l'une des revendications précédentes,
dans lequel la conductivité électrique est, pour une valeur de seuil inférieure de température, au moins de 10^-8 S/m et/ou pour une valeur de seuil supérieure de température, au maximum de 10^-4 S/m.

6. Boîtier (10) d'émetteur de rayons X à plusieurs tubes suivant l'une des revendications précédentes, dans lequel la première partie (17) du boîtier conductrice de l'électricité comporte du verre flint, du proceram, du nitrure de silicium, du carbure de silicium, de l'oxyde de zirconium, du silicium et/ou un matériau dopé.

7. Boîtier (10) d'émetteur de rayons X à plusieurs tubes suivant l'une des revendications précédentes,
dans lequel le boîtier (10) d'émetteur de rayons X à plusieurs tubes a une première douille (27) de réception d'un premier contact (15.1 ... 15.N) de haute tension et dans lequel la première douille (27) a un moyen (28) de fixation, en particulier un moyen de serrage, un moyen de vissage et/ou un moyen d'enfichage pour la connexion amovible d'une première électrode (16.1 ... 16.N) de haute tension au premier contact (15.1 ... 15.N) de haute tension.

8. Boîtier (10) d'émetteur de rayons X à plusieurs tubes suivant la revendication 7,
dans lequel, dans la première douille (27), est monté un passe-câble (29) isolant pour isoler électriquement de la première douille (27) l'électrode (16.1 ... 16.N) de haute tension pouvant être connectée.

9. Boîtier (10) d'émetteur de rayons X à plusieurs tubes suivant la revendication 7 ou 8,
dans lequel la première douille (27) est en un matériau ayant une conductivité électrique qui dépend de la température.

10. Boîtier (10) d'émetteur de rayons X à plusieurs tubes suivant l'une des revendications 7 à 9,
dans lequel un dispositif (33) d'étanchéité pour rendre étanche le boîtier (11) est disposé entre la première douille (27) et le boîtier (11).

11. Source (30) de rayons X à plusieurs tubes, comportant
- au moins un boîtier (10) d'émetteur de rayons X à plusieurs tubes suivant l'une des revendications précédentes,
- plusieurs tubes (31.1 ... 31.N) de rayons X,
dans laquelle chaque tube (31.1 ... 31.N) de rayons X comprend un boîtier sous vide ayant une cathode et une anode pour la production de rayonnement X, dans lequel une zone d'accélération d'électrons émis est prévue entre la cathode et l'anode et
dans laquelle les anodes ou les cathodes forment les électrodes (16.1 ... 16.N) de haute tension et sont connectées respectivement aux contacts (15.1 ... 15.N) de haute tension.

12. Source (30) de rayons X à plusieurs tubes suivant la revendication 11,
dans laquelle au moins l'un des tubes (31.1 ... 31.N) de rayons X fait saillie du boîtier (11), de manière à ce que du rayonnement X produit à l'anode se propage en s'éloignant du boîtier (11).

13. Source (30) de rayons X à plusieurs tubes suivant l'une des revendications 11 ou 12,
dans laquelle une distance entre la première partie (17) du boîtier conductrice de l'électricité et l'un des tubes (31.1 ... 31.N) de rayons X est plus petite qu'une étendue d'isolation se trouvant entre eux le long du milieu (13) de refroidissement isolant électriquement.

14. Dispositif (40) de rayons X, comportant
- une source (30) de rayons X à plusieurs tubes suivant l'une des revendications 11 à 13 et
- un détecteur (41) de rayons X.

15. Dispositif de tomodensitométrie assisté par ordinateur, comportant
- plusieurs dispositifs (40) de rayons X suivant la revendication 14, dans lequel les boîtiers (11) des boîtiers (10) d'émetteur de rayons X à plusieurs tubes sont constitués en forme d'arc de cercle, dans lequel les boîtiers (11) en forme d'arc de cercle recouvrent au moins 180° et dans lequel des tubes (31.1 ... 31.N) de rayons X voisins sont équidistants.
